# EUROPÄISCHE PATENTSCHRIFT

(11) **EP 1 932 902 B1**
(45) Veröffentlichungstag und Bekanntmachung des Hinweises auf die Patenterteilung: **24.06.2009**
(21) Anmeldenummer: 07122523.9
(22) Anmeldetag: 06.12.2007
(51) Int. Cl.: C12M 1/107

(54) **Gär- bzw. Faulbehälter**
Fermentation or digestion tank
Récipient de fermentation ou de digestion

(30) Priorität: 12.12.2006 DE 102006058932
(43) Veröffentlichungstag der Anmeldung: 18.06.2008
(73) Patentinhaber: Karl Buschmann Maschinenbau GmbH, 46499 Hamminkeln (DE)
(72) Erfinder:
(74) Vertreter: Mederle-Hoffmeister, Stefan

(56) Entgegenhaltungen:
- EP-A- 0 044 948
- DE-A1- 19 824 867
- DE-U1- 20 312 688
- US-A- 4 532 042
- US-A- 4 735 724

## Beschreibung

Die Erfindung betrifft einen Gär- bzw. Faulbehälter, insbesondere für Biogas-Anlagen bzw. biologische Kläranlagen gemäß dem Oberbegriff des Patentanspruchs 1.

Die DE-U-203 12 688 zeigt eine Vorrichtung zum Einspritzen einer im wesentlichen flüssigen Substanz in einen Gär- oder Faulbehälter. Dabei wird mittels einer Pumpe im Gärbehälter befindliche Substanz abgesaugt und über eine außerhalb des Gärbehälters liegende Zuführleitung, in welcher sich die Pumpe befindet, nahe des Füllstandes oder oberhalb des Füllstandes wieder in den Gärbehälter eingespritzt. Dadurch können Schwimm- und Deckschichten aufgebrochen oder aufgelöst werden und die Substanz im Gärbehälter in Bewegung gesetzt werden. Zur Durchmischung der Substanz im Gärbehälter befindet sich nahe des Bodens des Behälters ein Rührpropeller.

Die DE-A-198 24 867 beschreibt einen Reaktor zur Vergärung biogener Stoffe mit im Gärbehälter angeordneten Schächten zur Erzeugung einer Umlaufströmung. An den Gärbehälter ist außen eine mit einer Umwälzpumpe versehene Umlaufleitung angeschlossen, über welche im Gärbehälter befindliche Suspension in einem äußeren Bereich angesaugt wird und von unten in die sich in der Mitte des Behälters befindlichen Schächte gefördert wird.

Die US-A-4 735 724 offenbart einen anaeroben Faulbehälter für festes biologisch abbaubares Material, bei welchem in einem oberen Bereich festes Material eingebracht wird, und in einem unteren Bereich flüssiges Material abgezogen wird. Das Abflussmaterial kann über eine außerhalb des Behälters liegende Leitung, welche eine Pumpe und Ventile enthält, abgezogen und wieder in den oberen Behälterbereich eingespritzt werden.

Die EP-A-0 044 948 beschreibt eine Vorrichtung zur Erzeugung von Methangas aus landwirtschaftlichen Abfallstoffen mittels anaerober Bakterien. Zur Durchmischung der Stoffe im Reaktionsbehälter und um gegebenenfalls eine Schwimmschlammdecke aufzubrechen wird über eine externe Leitung 15 Material abgepumpt und über eine Druckleitung und Injektordüse wieder zugeführt. Die Menge des abgepumpten Materials kann über Ventile an der Entnahme- und Zufuhrstelle aus/in den Behälter geregelt werden.

Aus der US-A-4 532 042 ist eine Vorrichtung zur anaeroben Zersetzung flüssiger organischer Verbindungen bekannt. Über eine externe Leitung wird im oberen Bereich des Reaktionsbehälters Material abgepumpt und in den unteren Bereich wieder eingeführt, um eine bessere Durchmischung zu erreichen.

Aus der DE 94 04 188 U1 ist eine Mischeinrichtung für flüssigkeitsgefüllte Behälter bekannt. Diese Mischeinrichtung umfasst unter anderem ein mit einer Führungseinheit fest verbundenes Mischelement, das mittels einer Hebevorrichtung in den Behälter abzusenken und aus diesem zu heben ist. Ferner weist sie eine dem Mischelement und dessen Führungseinheit zugeordnete Führungs- und Halterungsvorrichtung auf, die im Wesentlichen aus einem auf dem Behälterboden angeordneten, im Bereich seiner Enden befestigten Führungsrohr und aus einer am Führungsrohr befestigten Auflage für das Mischelement und/oder dessen Führungseinheit gebildet wird. Am Rand des Behälters ist ein begehbares Podest angeordnet, an dessen vom Rand des Behälters abgewandter Vorderseite das Führungsrohr befestigt ist. Der Abstand zwischen dem Führungsrohr und der Wand des Behälters ist so bemessen, dass das Mischelement die für seine ungestörte Funktion notwendigen saugseitigen radialen Freiräume besitzt.

Eine derartige Fördereinrichtung, bei welcher das Mischelement aus einer Hebevorrichtung in den Behälter abgesenkt wird, ist jedoch konstruktiv relativ aufwendig und dementsprechend personal- und wartungsintensiv.

Weiterhin ist aus der DE 197 56 485 A1 ein Gärbehälter mit einer runden Bodenfläche und einem Füllstutzen sowie einer am Umfang des Gärbehälters angebrachten Fördereinrichtung in Form eines Rührwerks mit einer Antriebsachse bekannt. Das Rührwerk ist in einem unterhalb des Füllstutzens angeordneten Rührrohr angeordnet und die verlängerte horizontale Projektion der Antriebsachse schneidet den Umfang des Gärbehälters sekantenförmig.

Durch das Rührwerk wird für eine Durchmischung der Gärstoffe im Gärbehälter gesorgt. Das Rührwerk setzt sich aus einem Motor, einer Antriebsachse bzw. einer Antriebswelle und einem Förderelement in Form eines Rührkopfes, welcher gemäß der DE 197 56 485 A1 schrauben- oder propellerförmig ausgebildet sein kann, zusammen. Während sich der Motor außerhalb des Gärbehälters befindet, ist der Rührkopf innerhalb eines Reaktionsraums des Gärbehälters angeordnet. Die Wirkverbindung zwischen Motor und Rührkopf wird über eine Antriebsachse bzw. Antriebswelle sichergestellt, welche in einer korrespondierenden Durchführung durch die Behälterwand des Gärbehälters hindurch geführt ist. Im Bereich der Behälterwand ist es notwendig, die Antriebswelle zu lagern. Gerade im Bereich der Lager treten häufig Defekte bzw. Verschleißerscheinungen auf, sodass es nötig ist, die Lager zu wechseln bzw. das gesamte Rührwerk aus dem Behälter zu entfernen. Dazu muss der Behälter jedes Mal vollständig entleert werden, was für hohe Betriebskosten und einen unerwünschten Produktionsausfall sorgt.

Aufgabe der vorliegenden Erfindung ist es demnach, einen Gär- bzw. Faulbehälter anzugeben, bei welchem ein Austausch der Fördereinrichtung, insbesondere des Förderelements ohne eine Entleerung des entsprechenden Gär- bzw. Faulbehälters erfolgen kann.

Diese Aufgabe wird erfindungsgemäß durch einen Gär- bzw. Faulbehälter gemäß Patentanspruch 1 gelöst.

Es sei an dieser Stelle angemerkt, dass, wenn in der Folge ein Gärbehälter genannt ist, es sich in der Terminologie der vorliegenden Anmeldung immer um einen Gär- bzw. Faulbehälter handelt. Entsprechend handelt es sich bei der Nennung von Gärstoffen immer um Gär- bzw. Faulstoffe und bei einem Gärstoffkreislauf immer um einen Gär- bzw. Faulstoffkreislauf.

Weitere Merkmale der Erfindung sind in den Unteransprüchen enthalten.

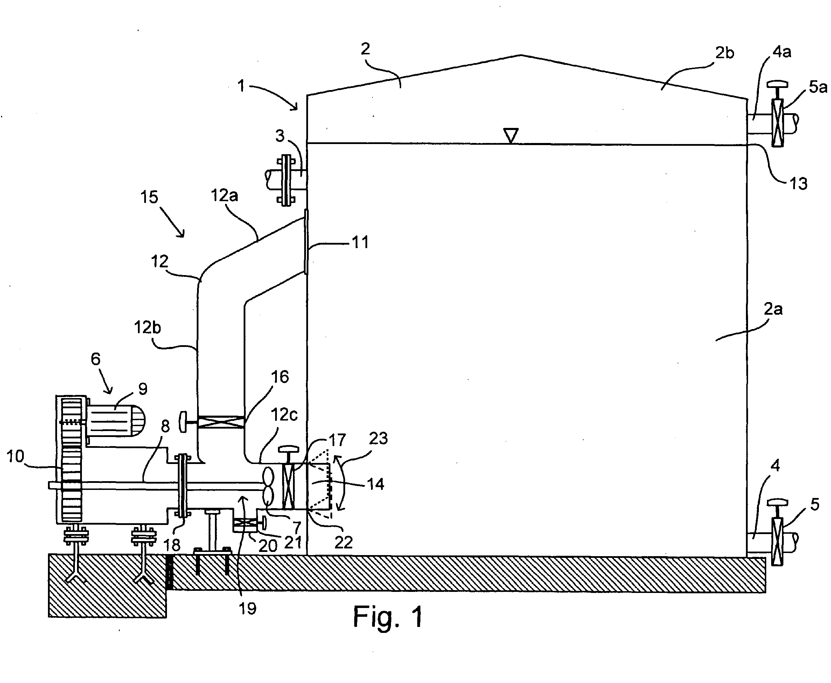

Die Erfindung wird im Folgenden mit Bezug auf die Zeichnung anhand einer bevorzugten Ausführungsform beispielhaft beschrieben. Als einzige Zeichnung zeigt
- Fig. 1: einen Querschnitt durch die bevorzugte Ausführungsform eines erfindungsgemäßen Gärbehälters.

Die bevorzugte Ausführungsform des erfindungsgemäßen Gär- bzw. Faulbehälters 1 umfasst einen Prozessraum 2, in welchem die Biogas-Erzeugung stattfindet, sowie einen Einlass 3, welcher zur Befüllung, und einen Auslass 4, welcher zur Entleerung des Gärbehälters 1 dient. Am Auslass 4 ist eine Absperrvorrichtung 5 in Form eines Schiebers angeordnet, welche eine unerwünschte Entleerung des Behälters verhindert. Alternativ zu einem Schieber kann die Absperrvorrichtung 5 auch in Form eines Ventils beispielsweise in Form eines Kugelventils oder eines Klappenventils vorliegen. Der Schieber ist manuell betätigbar, wobei auch eine automatisierte Betätigung auf elektrischem, pneumatischem oder hydraulischem Wege denkbar ist. Der Prozessraum 2 ist bis zu einem Betriebsfüllstand 13 mit zu vergärenden bzw. faulenden Stoffen 2 a gefüllt, während oberhalb des Betriebsfüllstands 13 das anfallende Gas in einem Gasraum 2 b gesammelt und über einen Gasauslass 4 a seiner Verwendung zugeführt wird. Der Gasauslass 4 a weist eine gasdichte Absperrvorrichtung 5 a auf. Bei dem Behälter 1 kann es sich um einen Blechstau- einen Kunststoff- oder einen gemauerten Behälter handeln, welcher ggf. eine entsprechende gasdichte Auskleidung besitzt. Im Bereich gemauerter Behälter kommen insbesondere Behälter aus Beton, welche entsprechende Kernbohrungen als Öffnungen aufweisen, in Frage.

Der Gärbehälter 1 weist weiterhin eine Misch- bzw. Fördereinrichtung 6 auf, welche ein Misch- bzw. Förderelement in Form eines Propellers 7, eine Antriebswelle 8 sowie eine Antriebseinheit in Form eines Motors 9 umfasst. Der Propeller 7 steht über die Antriebswelle 8 mit dem Motor 9 kraftschlüssig in Wirkeingriff. In der beschriebenen bevorzugten Ausführungsform erfolgt der Kraftübertrag zwischen Motor 9 und Antriebswelle 8 über ein Getriebe 10. Alternativ hierzu ist selbstverständlich jegliche andere Antriebsmethode, insbesondere ein Ketten- oder Riemenantrieb, denkbar.

Es sei an dieser Stelle angemerkt, dass an Stelle eines Propellers 7 als Förderelement selbstverständlich auch eine Schraube (beispielsweise eine schiffschraubenartig ausgebildete Schraube) oder dergleichen Vorrichtung denkbar ist.

Durch die Fördereinrichtung 6 wird ein Gär- bzw. Faulstoff-Kreislauf definiert, welcher der gleichmäßigen Verteilung der Gär- bzw. Faulstoffe, welche insbesondere Abfallstoffe und nachwachsende Rohstoffe umfassen können, und einer kontinuierlichen Entgasung derselben bzw. des Gärbehälters 1 dient. Gär- bzw. Faulstoffe werden dem Prozessraum 2 über den Einlass 3, welchem ein Flansch zur sicheren Einleitung derselben zugeordnet ist, zugeführt und sollen dann sowohl in horizontaler als auch in vertikaler Richtung mit dem bereits im Behälter befindlichen Substrat vermengt bzw. vermischt werden.

Die Vermischung erfolgt mittels der Fördereinrichtung 6, welche zumindest teilweise die am Einlass 3 eintretenden Gärstoffe über eine Öffnung 11 in ein mit dieser Öffnung in Fluidverbindung stehendes Rohr 12 saugt. Die Öffnung 11 ist in einem oberen Bereich des Gärbehälters 1 bzw. des Prozessraums 2 (der Mittelpunkt der Öffnung befindet sich auf Höhe von 80 % - 90 % des Betriebsfüllstandes 13 des Gärbehälters 1) in der Nähe des Einlasses 3 angeordnet. Das Rohr 12 ist in etwa n-förmig ausgebildet, wobei der nach oben gewandte Schenkel 12 a des Rohres 12 mit dem senkrechten Abschnitt 12 b des Rohres 12 einen Winkel von größer 90° (in etwa 100° bis 110°) einschließt. Der Gärstoff, welcher durch die Öffnung 11 eingesaugt wird, wird über einen unteren Schenkel 12 c des Rohres 12, in welchem der Propeller 7 angeordnet ist, über diesen in eine im Gärbehälter angeordnete Öffnung 14, in welcher der untere Schenkel 12 c des Rohres 12 hineinragt, eingespeist.

Der untere Schenkel 12 c des Rohres 12 ist in der Öffnung 14 im Behälter 1 mittels eines Kugelgelenks 22 beweglich bzw. schwenkbar gelagert. Dadurch ist der Winkel, unter dem die Gär- bzw. Faulstoffe bezüglich des Behälterbodens eingeleitet werden, variabel, sodass alle gewünschten Strömungsverhältnisse herstellbar sind. An Stelle des Kugelgelenks 22 sind auch eine entsprechende Blendenanordnung, Düsenanordnung oder jegliche andere die Strömung leitende Vorrichtung denkbar. Dadurch entsteht die gewünschte Durchmischung der Gärstoffe in einem Kreislauf, welcher von der Öffnung 11 über das Rohr 12 hin zur Öffnung 14 und dort über eine Zirkulation bzw. Strömung im Prozessraum 2 wieder zur Öffnung 11 führt. Der Gärbehälter 1 weist also in anderen Worten gesagt einen außerhalb des Prozessraums 2 angeordneten Abschnitt 15 des Gärstoff-Kreislaufs auf, welcher im Wesentlichen durch das Rohr 12 sowie Teile der Fördereinrichtung 6 (Teile der Antriebswelle 8 sowie insbesondere den Propeller 7) definiert wird, auf. Der Mittelpunkt der Öffnung 14 befindet sich auf Höhe von 5 % - 20 % des Betriebsfüllstandes 13 des Gärbehälters 1.

Die Fördereinrichtung 6 ist teilweise, sowohl in Form des Propellers 7 als auch in Form eines Abschnitts der Antriebswelle 8 in dem außerhalb des Prozessraums 2 angeordneten Kreislaufabschnitt 15, nämlich im Schenkel 12 c des Rohres 12, angeordnet. Bei alternativen bevorzugten Ausführungsformen können analog zur hier beschriebenen bevorzugten Ausführungsform beispielsweise anstelle des Propellers 7 als Misch- bzw. Förderelement eine Schraube, insbesondere eine dreiblättrige Schraube (ähnlich einer Schiffschraube) im Kreislaufabschnitt 15 angeordnet sein. Alternativ ist es auch denkbar, dass die gesamte Fördereinrichtung 6 im Kreislaufabschnitt 15 angeordnet ist. Alternativ zur Anordnung im Schenkel 12 c kann die Fördereinrichtung 6 bzw. können Teile der Fördereinrichtung 6 auch in anderen Bereichen des Rohres 12 angeordnet sein (insbesondere im Schenkel 12 a oder im Abschnitt 12 b).

Im Rohr 12 sind in einem dem unteren Schenkel 12 c zugewandten Bereich des senkrechten Abschnitts 12 b des Rohres 12 sowie in einem der Öffnung 14 zugewandten Bereich des unteren Schenkels 12 c des Rohres 12 zwei Absperreinrichtungen 16, 17 in Form von Schiebern, insbesondere Plattenschiebern, Doppelplattenschiebern oder Brillenschiebern angeordnet, durch deren Schließen es ermöglicht wird, die Fördereinrichtung 6, insbesondere das Förderelement zu reparieren bzw. dieselbe bzw. dasselbe auszuwechseln, während der Prozessraum 2 des Gärbehälters 1 mit Gärstoffen bzw. Substrat gefüllt bleiben kann. Auch hier wären als Alternativen zu Schiebern Ventile, insbesondere Kugelventile bzw. Klappenventile mit einer oder mehreren Klappen denkbar.

Die Antriebsvorrichtung (Motor 9), welche die Antriebswelle 8 antreibt, ist über einen Flansch 18 an das Rohr 12 angeflanscht. Im Bereich des Flansches 18 ist die Antriebswelle 8 mittels geeigneter Lager, beispielsweise Rollen- bzw. Kugellager gelagert und mittels einer Dichtung fluiddicht gegen die Umgebung bzw. gegen den Motor 9 abgedichtet. Sowohl der Motor 9 als auch der außen liegende Kreislaufabschnitt sind fest im Boden verankert. Hierzu finden Verankerungselemente in Verbindung mit passenden Stützen Verwendung.

Für eine nötige Entleerung eines Volumens 19, welches im Rohr 12 durch die beiden Absperrvorrichtungen 16, 17 definiert ist, weist der untere Schenkel 12 c des Rohres 12 einen Auslass 20 auf, welcher mittels einer Absperrvorrichtung 21, die in der hier beschriebenen bevorzugten Ausführungsform in Form eines Schiebers vorliegt, verschlossen ist. Bei einem Austausch bzw. einer Reparatur der Fördereinrichtung 6 bzw. des Förderelements können die im Volumen 19 befindlichen Gärstoffe durch den Auslass 20 abgelassen werden. Der Auslass 20 ist in etwa an der am tiefsten liegenden Stelle des außen liegenden Kreislaufabschnitt 15 bzw. an der tiefstliegenden Stelle des Volumens 19 angebracht ist, um eine sichere Entleerung bei einer Reparatur der Fördereinrichtung 6 gewährleisten zu können. Auch die Absperrvorrichtung 21 liegt in der bevorzugten Ausführungsform in Form eines Schiebers vor, wobei hier an Stelle eines Schiebers jegliche andere Absperrvorrichtung, insbesondere in Form eines Ventils, beispielsweise eines Kugelventils oder Klappenventils, denkbar wäre. Alternativ oder zusätzlich zur Absperrvorrichtung 21 kann der Auslass 20 mit einer Verschlusskappe, welche fluiddicht schließt, versehen sein.

Es sei an dieser Stelle angemerkt, dass sämtliche Absperreinrichtung (Absperreinrichtungen 5, 16, 17, 21) teilweise, d.h. an den Teilen, an denen sie mit den Gärstoffen in Verbindung kommen, mit einem Korrosionsschutz in Form einer keramischen Beschichtung versehen sind. Alternativ hierzu kommt selbstverständlich auch eine Herstellung der mit den Gärstoffen in Verbindung stehenden Teile der Absperrvorrichtung aus einem korrosionsbeständigen Material, beispielsweise einer Keramik oder geeigneten Stählen, insbesondere Edelstählen oder Kunststoffen, in Frage. Auch eine Herstellung der gesamten Absperreinrichtung(en) 5, 16, 17, 21 aus korrosionsbeständigem Material bzw. eine vollständige Beschichtung der Absperreinrichtungen 5, 16, 17, 21 ist denkbar.

Es bleibt noch anzumerken, dass zur einfachen Entleerung des Volumens 19 die Absperreinrichtung 16 oberhalb bzw. stromaufwärts des Propellers 7 angeordnet ist, während die Absperreinrichtung 17 stromabwärts des Propellers 7 angeordnet ist. Die Begriffe "stromaufwärts" und "stromabwärts" beziehen sich dabei auf den normalen Betrieb der Anlage (entgegen dem Uhrzeigersinn), wobei angemerkt sei, dass die Anlage auch im umgekehrten Sinne (im Uhrzeigersinn) betrieben werden kann. Dies ist insbesondere notwendig, um eventuelle Blockaden bzw. Verstopfungen im Rohr 12 zu beseitigen. Auch für eine bessere Gasabfuhr kommt ein Betrieb entgegen der herkömmlichen Richtung in Frage.

Die Absperrvorrichtungen 5, 16, 17, 21 sind in der bevorzugten Ausführungsform alle manuell bedienbar, wobei alternativ oder zusätzlich bei allen Absperrvorrichtungen eine elektrische bzw. pneumatische bzw. hydraulische Betätigung in Frage kommt. Insbesondere eine pneumatische bzw. eine hydraulische Betätigung empfiehlt sich auf Grund der notwendigen erhöhten Sicherheitsmaßnahmen beim Umgang mit (leicht entzündlichen) Biogasen. Bei einer gleichzeitigen Möglichkeit zur händischen Bedienung ist auch für den Notfall, in dem die automatische Bedienung versagt, für einen sicheren Betrieb der Anlage gesorgt.

An Stelle einer Kraftübertragung über einen Riementrieb oder ein Getriebe mit einer insbesondere ununterbrochene Antriebswelle 8 ist auch ein Antrieb, welcher eine Magnetkupplung aufweist und somit auf eine entsprechende Lagerung im Flansch 18 verzichten kann, denkbar.

Ferner sind an Stelle röhrenförmiger Elemente (Rohr 12) teilweise oder insgesamt auch schlauchartige Elemente denkbar, welche über geeignete Verbindungsmittel, insbesondere Flansche mit dem Gärbehälter bzw. entsprechenden Rohren in Fluidverbindung stehen.

## Patentansprüche

1. Gär- bzw. Faulbehälter (1), insbesondere für Biogas-Anlagen bzw. biologische Kläranlagen, mit einem Prozessraum (2), einem Einlass (3) und einem Auslass (4) sowie einer Fördereinrichtung (6) und einem Gär- bzw. Faulstoff-Kreislauf, in welchem in dem Gär- bzw. Faulbehälter befindliche Gär- bzw. Faulstoffe durch die Fördereinrichtung (6) zirkuliert werden,
wobei der Gär- bzw. Faulbehälter (1) einen außerhalb des Prozessraums angeordneten Kreislaufabschnitt (15) umfasst, und
die Fördereinrichtung (6) wenigstens teilweise in dem außerhalb des Prozessraums angeordneten Kreislaufabschnitt (15) angeordnet ist,
**dadurch gekennzeichnet,**
**dass** die Fördereinrichtung ein Rührwerk umfasst.

2. Gär- bzw. Faulbehälter (1) nach Anspruch 1,
**dadurch gekennzeichnet,**
**dass** der Kreislaufabschnitt (15) durch eine Fluidverbindung zwischen einem oberen Bereich und einem unteren Bereich des Gär- bzw. Faulbehälters (1) gebildet ist.

3. Gär- bzw. Faulbehälter (1) nach einem der vorhergehenden Ansprüche,
**dadurch gekennzeichnet,**
**dass** der Kreislaufabschnitt wenigstens teilweise durch in etwa röhrenförmige Elemente, insbesondere ein in etwa n-förmiges Rohrstück (12) gebildet ist.

4. Gär- bzw. Faulbehälter (1) nach einem der vorhergehenden Ansprüche,
**dadurch gekennzeichnet,**
**dass** der Kreislaufabschnitt (15) wenigstens teilweise durch flexible, insbesondere schlauchartige Elemente gebildet ist.

5. Gär- bzw. Faulbehälter (1) nach einem der vorhergehenden Ansprüche,
**dadurch gekennzeichnet,**
**dass** der Kreislaufabschnitt (15) eine Öffnung, insbesondere einen Flansch (18) aufweist, an welcher die Fördereinrichtung (6) angeordnet, insbesondere angeflanscht ist, wobei die Fördereinrichtung (6) wenigstens teilweise in den Kreislaufabschnitt (15) hineinragt.

6. Gär- bzw. Faulbehälter (1) nach einem der vorhergehenden Ansprüche,
**dadurch gekennzeichnet,**
**dass** der Kreislaufabschnitt (15) einen Auslass (20) aufweist, welcher insbesondere an einer tief liegenden Stelle, weiterhin insbesondere an der am tiefsten liegenden Stelle des Kreislaufabschnitts (15) angeordnet ist.

7. Gär- bzw. Faulbehälter (1) nach Anspruch 6,
**dadurch gekennzeichnet,**
**dass** im Bereich des Auslasses (20) eine Verschluss- bzw. Absperreinrichtung (21), insbesondere ein Plattenschieber, Doppelplattenschieber oder Brillenschieber oder ein Ventil, insbesondere Kugel- oder Klappenventil bzw. eine Verschlusskappe angeordnet ist.

8. Gär- bzw. Faulbehälter (1) nach einem der vorhergehenden Ansprüche,
**dadurch gekennzeichnet,**
**dass** in dem Kreislaufabschnitt (15) wenigsten eine, insbesondere zwei Absperreinrichtungen (16, 17) angeordnet ist/sind.

9. Gär- bwz. Faulbehälter (1) nach einem der vorhergehenden Ansprüche, insbesondere nach Anspruch 8,
**dadurch gekennzeichnet,**
**dass** in dem Kreislaufabschnitt (15) zwei Absperreinrichtungen (16, 17) angeordnet sind, wobei eine der Absperreinrichtungen (16) stromaufwärts und eine der Absperreinrichtungen (17) stromabwärts von der Fördereinrichtung (6) angeordnet ist.

10. Gär- bzw. Faulbehälter (1) nach einem der vorhergehenden Ansprüche,
**dadurch gekennzeichnet,**
**dass** die Fördereinrichtung (6) eine Pumpe umfasst.

11. Gär- bwz. Faulbehälter (1) nach einem der vorhergehenden Ansprüche,
**dadurch gekennzeichnet,**
**dass** die Fördereinrichtung ein Förderelement (7) umfasst, das über eine Antriebswelle (8) mit einer Antriebsvorrichtung (9) in Wirkeingriff steht.

12. Gär- bwz. Faulbehälter (1) nach einem der vorhergehenden Ansprüche,
**dadurch gekennzeichnet,**
**dass** das Förderelement einen Propeller (7) oder eine Schraube oder ein Förderelement einer Pumpe, insbesondere eine Schnecke bzw. eine Kreiselpumpe umfasst.

## Claims

1. Fermentation or digestion tank (1), in particular for biogas plants or biological clarification plants, having a process space (2), an inlet (3) and an outlet (4) and a conveyor (6) and a fermentation-material or digestion-material circuit, in which fermentation or digestion materials located in the fermentation or digestion tank are circulated by way of the conveyor (6), the fermentation or digestion tank (1) comprising a circuit portion (15) arranged outside the process space, and the conveyor (6) being arranged, at least in part, in the circuit portion (15) arranged outside the process space, **characterized in that** the conveyor comprises an agitator.

2. Fermentation or digestion tank (1) according to Claim 1, **characterized in that** the circuit portion (15) is formed by a fluid connection between a top region and a bottom region of the fermentation or digestion tank (1).

3. Fermentation or digestion tank (1) according to one of the preceding claims, **characterized in that** the circuit portion is formed, at least in part, by approximately tubular elements, in particular an approximately n-shaped pipe component (12).

4. Fermentation or digestion tank (1) according to one of the preceding claims, **characterized in that** the circuit portion (15) is formed, at least in part, by flexible, in particular hose-like elements.

5. Fermentation or digestion tank (1) according to one of the preceding claims, **characterized in that** the circuit portion (15) has an opening, in particular a flange (18), on which the conveyor (6) is arranged, in particular flanged, the conveyor (6) projecting, at least in part, into the circuit portion (15).

6. Fermentation or digestion tank (1) according to one of the preceding claims, **characterized in that** the circuit portion (15) has an outlet (20) which is arranged, in particular, at a low-level location, furthermore, in particular, at the lowest-level location of the circuit portion (15).

7. Fermentation or digestion tank (1) according to Claim 6, **characterized in that** a closure or shut-off means (21), in particular a parallel slide gate valve, double disc parallel slide gate valve or spectacle-type slide valve, or a valve, a ball valve or flat valve in particular, or a closure cap is arranged in the region of the outlet (20).

8. Fermentation or digestion tank (1) according to one of the preceding claims, **characterized in that** at least one shut-off means is, in particular two shut-off means (16, 17) are, arranged in the circuit portion (15).

9. Fermentation or digestion tank (1) according to one of the preceding claims, in particular according to Claim 8, **characterized in that** two shut-off means (16, 17) are arranged in the circuit portion (15), one of the shut-off means (16) being arranged upstream and one of the shut-off means (17) being arranged downstream, of the conveyor (6).

10. Fermentation or digestion tank (1) according to one of the preceding claims, **characterized in that** the conveyor (6) comprises a pump.

11. Fermentation or digestion tank (1) according to one of the preceding claims, **characterized in that** the conveyor comprises a conveying element (7) which is in operative engagement with a drive device (9) via a drive shaft (8).

12. Fermentation or digestion tank (1) according to one of the preceding claims, **characterized in that** the conveying element comprises a propeller (7) or a screw or a delivery element of a pump, in particular a worm or a centrifugal pump.

## Revendications

1. Récipient de fermentation ou de digestion (1), en particulier pour des installations de biogaz ou des installations d'épuration biologiques, avec une chambre de traitement (2), une entrée (3) et une sortie (4) ainsi qu'un dispositif de transport (6) et un circuit de matières de fermentation ou de digestion, dans lequel les matières de fermentation ou de digestion se trouvant dans le récipient de fermentation ou de digestion sont mises en circulation par le dispositif de transport (6), dans lequel le récipient de fermentation ou de digestion (1) présente une partie de circuit (15) disposée à l'extérieur de la chambre de traitement, et le dispositif de transport (6) est disposé au moins en partie dans la partie de circuit (15) disposée à l'extérieur de la chambre de traitement, **caractérisé en ce que** le dispositif de transport comprend un mécanisme d'agitation.

2. Récipient de fermentation ou de digestion (1) selon la revendication 1, **caractérisé en ce que** la partie de circuit (15) est formée par une liaison fluide entre une zone supérieure et une zone inférieure du récipient de fermentation ou de digestion (1).

3. Récipient de fermentation ou de digestion (1) selon l'une quelconque des revendications précédentes, **caractérisé en ce que** la partie de circuit est formée au moins en partie par des éléments sensiblement tubulaires, en particulier par une pièce tubulaire sensiblement en forme de n (12).

4. Récipient de fermentation ou de digestion (1) selon l'une quelconque des revendications précédentes, **caractérisé en ce que** la partie de circuit (15) est formée au moins en partie par des éléments flexibles, en particulier sous forme de tuyaux flexibles.

5. Récipient de fermentation ou de digestion (1) selon l'une quelconque des revendications précédentes, **caractérisé en ce que** la partie de circuit (15) présente une ouverture, en particulier une bride (18), sur laquelle le dispositif de transport (6) est disposé, en particulier bridé, le dispositif de transport (6) pénétrant au moins en partie dans la partie de circuit (15).

6. Récipient de fermentation ou de digestion (1) selon l'une quelconque des revendications précédentes, **caractérisé en ce que** la partie de circuit (15) présente une sortie (20), qui est disposée en particulier en un point situé en profondeur, et en particulier au point le plus profond de la partie de circuit (15).

7. Récipient de fermentation ou de digestion (1) selon la revendication 6, **caractérisé en ce qu'**un dispositif de fermeture ou d'arrêt (21), en particulier une vanne à plaques parallèles, une vanne à double plaque ou un aiguillage à deux directions, ou une soupape, en particulier une soupape à bille ou une soupape à clapet ou un capuchon de fermeture, est disposé dans la région de la sortie (20).

8. Récipient de fermentation ou de digestion (1) selon l'une quelconque des revendications précédentes, **caractérisé en ce qu'**au moins un, de préférence deux, dispositif(s) d'arrêt (16, 17) est/sont disposé(s) dans la partie de circuit (15).

9. Récipient de fermentation ou de digestion (1) selon l'une quelconque des revendications précédentes, en particulier selon la revendication 8, **caractérisé en ce que** deux dispositifs d'arrêt (16, 17) sont disposés dans la partie de circuit (15), un des dispositifs d'arrêt (16) étant disposé en amont et un des dispositifs d'arrêt (17) étant disposé en aval du dispositif de transport (6).

10. Récipient de fermentation ou de digestion (1) selon l'une quelconque des revendications précédentes, **caractérisé en ce que** le dispositif de transport (6) comprend une pompe.

11. Récipient de fermentation ou de digestion (1) selon l'une quelconque des revendications précédentes, **caractérisé en ce que** le dispositif de transport comprend un élément de transport (7), qui est en prise active avec un dispositif d'entraînement (9) au moyen d'un arbre d'entraînement (8).

12. Récipient de fermentation ou de digestion (1) selon l'une quelconque des revendications précédentes, **caractérisé en ce que** l'élément de transport comprend une hélice (7) ou une vis ou un élément de transport d'une pompe, en particulier une vis sans fin ou une pompe centrifuge.
